# EUROPEAN PATENT APPLICATION

(11) **EP 1 530 047 A1**
(43) Date of publication of application: **11.05.2005**
(21) Application number: 03025651.5
(22) Date of filing: 07.11.2003
(51) Int. Cl.: G01N 33/68, G01N 33/566

(54) **Proximal markers of arterial thrombosis and inflammation for risk stratification of coronary heart disease**

(71) Applicant: Roche Diagnostics GmbH, 68305 Mannheim (DE); F. Hoffmann-La Roche AG, 4070 Basel (CH)
(72) Inventor: Lehmann, Hans-Peter, Dr., 82387 Antdorf (DE); Cant, Charles, Dr., 81476 München (DE); Hallermayer, Klaus, Dr., 82340 Feldafing (DE)
(74) Representative: Huhn, Michael, Dr.

(57) **Abstract**

The present invention relates to a method for establishing a diagnosis if an individual has suffered from or a prognosis if an individual is at risk of suffering from a coronary heart event, which method comprises
- determining the concentration of sCD40L in a body fluid of the individual;
- determining the concentration of a proximal inflammatory marker, preferably PAPP-A or Lp-PLA2
- comparing the values obtained with reference values; and
- establishing the diagnosis or the prognosis in respect to the cardiovascular event.

The combination according to the invention can be used with further markers selected from further inflammatory markers, anti-inflammatory markers and/or neurohumoral markers.

## Description

The present invention relates to combinations of particular diagnostic markers for the risk determination of coronary heart disorders. More precisely, the combinations according to the present invention allow establishing a more precise prediction for which coronary heart event the patient tested is at risk.

Coronary heart diseases remain the most common cause of mortality in the western world. Several risk factors leading to various coronary heart disorders are known. One example is the level of total and of HDL cholesterol in the blood. However, the predictive value of a cholesterol level is low, and the identification of a large number of individuals having a high coronary heart event risk profile is not possible by measuring the cholesterol level.

Coronary heart disease is a general term including several coronary heart events which exhibit different risk rates for the patient requiring different treatments and, respectively, different grades of supervision of the individual under examination.

Individuals suffering from a coronary heart disease can be divided into individuals showing no clinical symptoms and those which appear with breathlessness and/or chest pain. The latter group can be divided into individuals having stable angina pectoris (SAP) and those with acute coronary syndromes (ACS). ACS patients can show unstable angina pectoris (UAP), or these individuals have already suffered from a myocardial infarction (MI). MI can be a ST-elevated MI or a non ST-elevated MI. The occurring of a MI can be followed by a left ventricular dysfunction (LVD). Finally, LVD patients undergo congestive heart failure (CHF) with a mortality rate of roughly 15 %, or they show no symptoms.

Patients who present with chest pain are examined for an ST-elevation or -lowering. If this is the case, he individual has suffered from a MI, with a probability of close to 100 % and will be hospitalized. As however not all individuals with a MI show ST-abnormalities, the cardiac troponin (cTnT or cTNI) level is determined indicating - in case of abnormally high values - a high probability that a MI has occurred.

The determination of troponin levels is established as being a valuable tool for the analysis of MI and has become a routine step for the physician.

However, seen the complexity of coronary diseases and the different stages an individual having such disease passes through before a congestive heart failure (CHF) occurs, physicians want a system which allows an early diagnosis of an individual at risk for any coronary heart event preceding AMI and CHF.

The person skilled in the art is aware of a large number of different molecular markers which may be useful for the diagnosis of a coronary heart disease or, respectively, subtypes thereof. Examples for such markers are the following:
Pregnancy-associated plasma protein A (PAPP-A); high sensitive C-reactive protein (hsCRP; placental growth factor (PIGF); interleukin-18 (IL-18/IL-18b); brain natriuretic peptide (BNP); NT-pro brain natriuretic peptide (NT-proNP); ischemic modified albumin (IMA), soluble CD40 Ligand (sCD40L), cardiac troponin I/T (cTnI/T), IL-10, ICAM-1, VCAM-1, E-selectin, P-selectin, IL-6, VEGF, Fibrinogen, Serum Amyloid A (SAA), CKMB, MPO, LpPLA2, GP-BB, IL1RA, TAFI, soluble fibrin, anti-oxLDL, MCP-1, procoagulant tissue factor (TF), MMP-9, Ang-2, IL-8, bFGF, von Willebrand Factor (vWF), VLDL, PAI-1

Some of the markers mentioned above are known and characterized, and their role in the pathophysiology of coronary diseases is established, contrary to others which have not been examined in detail in respect to their prognostic value.

Almost all markers cited beforehand are of diagnostic value in respect to certain coronary heart events. For example, markers like CRP are valuable for the diagnosis and prediction of an inflammation, which may lead to plaque rupture and MI.

Even though the diagnostic value of several markers is established, and some have been found to give highly important and exact diagnostic information relative to a certain coronary heart event, many markers can only be used alone or in combination with certain other markers. The use of a combination of markers has only restrictedly been described.

Peng et al. in Clinica Chimica Acta 319 (2002) 19-26, disclose the combination of sCD40L with sICAM-1 and sVCAM-1.
Blankenberg et al. in Circulation. 2002; 106:24-30, describe the combination of CRP and IL-6.
Autiero et al. in Journal of Thrombosis and Haemostasis, 1:1356-1370, describe the combination of PIGF and VEGF.

A considerable amount of markers give no additional information in combination with certain further markers, as their expressions in body fluids, in general blood, is not independent from each other. The production of a certain protein appropriate as a marker may trigger the production of other markers meaning that both markers indicate the same pathophysiological phenomenon. It is practically not established which markers or which subgroups of markers lend themselves for combinations giving a highly precise diagnostic information or which can be used to establish a coronary heart risk profile of a patient.

Thus, the object of the present invention is to find a general combination of diagnostic markers for coronary heart events which allow for a more precise diagnosis and prognosis which coronary heart event the individual under examination is likely to suffer from in the future. Furthermore, the invention should also allow the diagnosis prognosis of individuals at risk of CHD which do not show symptoms of acute disease. In particular the invention should allow the diagnosis of individuals at risk of CHD.

This object is attained by a method for establishing a diagnosis and prognosis if an individual is at risk of suffering from a future coronary heart event, which method comprises
- determining the concentration of sCD40L and a proximal inflammatory marker in a body fluid of the individual;
- comparing the values obtained with reference values; and
- establishing the diagnosis or the prognosis in respect to the said coronary heart event.

When in the context of the present invention reference is made to "coronary heart event", this refers to a particular pathophysiological state related to coronary heart diseases in general. Examples for coronary heart events are ACS, AMI (acute myocardial infarction), SAP, UAP, CHF, LVD, and further diseases which are known to the person skilled in the art. A list of the coronary heart events which can be diagnosed and/or predicted using the method according to the present invention will be given below.

The individual can be any individual presenting with chest pain or an individual who does not show any symptoms related to a coronary heart disease. For example, the individual may show no chest pain, and/or no ST-elevation.

The method according to the present invention is in particular advantageous for individuals at elevated risk which do not apparently show disease symptoms.

Patients at risk of CHD may include calculated risk scores according to Framingham, Procam and comparable risk scores, in particular patients with diabetes type I and II, diabetic patients with renal disorders, patients after an acute cardiovascular event, e.g. post myocardial infarction or ischemic stroke, patients with advanced and severe atherosclerosis, peripheral arteriovascular disease (PAD), hypercholesterolemia, hypertension, genetic predisposition (e.g. familial history of AMI and/or ischemic stroke), metabolic syndromes, obesity, ex-smokers, and other non-symptomatic patients without conventional risk factors.

The concentration of the proximal inflammatory marker can be determined before the concentration of sCD40L is determined. The determination of the proximal inflammatory marker can also occur simultaneously or after the determination of the sCD40L.

In a preferred embodiment of the present invention, the concentration of at least one marker further to sCD40L and the proximal inflammatory marker is determined, with the said further marker being an inflammatory marker.

In a further preferred embodiment of the present invention, the concentration of at least one marker further to sCD40L and the proximal inflammatory marker is determined, with the said further marker being an anti-inflammatory marker.

In a further preferred embodiment of the present invention, the concentration of at least one marker further to sCD40L and the proximal inflammatory marker is determined, with the said further marker being a neurohumoral marker.

In a further preferred embodiment of the present invention, the concentration of at least two markers further to sCD40L and the proximal inflammatory marker is determined, with one further marker being selected from anti-inflammatory markers and the other further marker being selected from neurohumoral markers.

It has been found that the specific marker combination according to the present invention, namely sCD40L and the proximal inflammatory marker, permits a reliable analysis of the pathophysiological coronary heart state of an individual.

The method according to the present invention lends itself for the diagnosis and prognosis of coronary heart events, as well as for therapy stratification and treatment monitoring.

"Diagnosis" in the context of the present invention refers to verifying if an individual has suffered from a certain coronary heart event.

"Prognosis" in the context of the present invention refers to the prediction probability (in %) an individual will suffer from a certain coronary heart event.

"Therapy stratification" in the context of the present invention refers to assessing the appropriate therapeutic treatment for the coronary heart event which may occur or has occurred.

"Treatment monitoring" in the context of the present invention refers to controlling and, optionally, adjusting the therapeutic treatment of an individual.

"Therapeutic treatment" includes any treatment which may alter the pathophysiological state of an individual, and includes, for example, administering of pharmaceutical drugs as well as surgical treatment (e.g. revascularization, balloon dilatation, stenting).

The combination according to the invention uses markers which each predict the risk for a certain cardiovascular event or certain coronary heart events, with the determination of each marker concentration giving an independent result and, thus, the predictability for each respective event being independent of the results of the other markers.

It has been established (see WO 03/040692 and Circulation 2001;104:2266-2268) that sCD40L can advantageously be used in combination with an inflammatory marker, in particular hsCRP. It has however nowhere been established that a combination of sCD40L with a proximal inflammatory marker gives the advantageous diagnostic results the inventors of the present invention have found.

A "cardiovascular event", as used in the context of the present invention, designates a certain pathophysiological coronary heart state which is not found in healthy individuals and which falls under the general term "cardiovascular disease". The person skilled in the art is aware of the various cardiovascular events which can occur in mammals, in particular humans.

The cardiovascular events which can be diagnosed or predicted using the marker combination(s) according to the present invention include the following: coronary heart disease (CHD); stable angina pectoris (SAP); unstable angina pectoris (UAP); acute coronary syndrome (ACS); acute myocardial infarction (AMI), both in cases of ST-elevation and non ST-elevation; left ventricular dysfunction (LVD); congestive heart failure (CHF).

sCD40L is in particular associated with platelet activation, platelet aggregation and thrombus propagation, representative of the risks that plaque having already become vulnerable will rupture, resulting in reversible vascular occlusion (UAP) or irreversible vascular occlusion (AMI) which may lead to left ventricular dysfunction (LVD), congestive heart failure (CHF) and death.

It has been recognized by the inventors of the present invention that sCD40L is the most important marker for diagnosis and prediction of coronary heart events in risk patients, besides TnT which is known to be of particular diagnostic value (laboratory gold standard for AMI) to the person skilled in the art. The predictive value of TnT is however limited to acute cardiovascular events, as it is indicative of an ischemic damage of heart tissue, e.g. myocyte necrosis.

sCD40L has been supposed to be a marker of inflammation, see Aukrust, Circulation 1999; 100: 604 - 620, and hence to indicate a risk for the occurrence of coronary heart events. In WO 03/040691 mentioned beforehand, sCD40L has been described as a systemic marker of inflammation. However, nowhere in WO 03/040691 it is mentioned that sCD40L cannot just be regarded as a systemic marker for coronary heart events, but as a proximal systemic marker for atherothrombotic events, i.e. platelet activation, as a result of arterial plaque erosion and rupture. Furthermore, nowhere in WO 03/040691 it is mentioned or suggested that sCD40L can advantageously be used in combination with a proximal inflammatory marker.

Proximal inflammatory markers are in particular associated with the risk that plaques already present in an individual will undergo inflammation, augmenting the probability of plaque rupture and thrombus formation.

Inflammatory markers known to the person skilled in the art include the following:
CRP, preferably hsCRP, fibrinogen, serum amyloid A (SAA), pregnancy-associated polypeptide A (PAPP-A), intercellular adhesion molecules (e.g. ICAM-1, VCAM-1), IL-1-beta, IL-6, IL-18/IL-18b; TNF-alpha; myeloperoxidase (MPO); TF; monocyte chemoattractant protein 1 (MCP-1); P-selectin; E-selectin; matrix metalloproteinases (MMPs, e.g. MMP-1, -2, -3, -4, -5, -6, -7, -9, -10, -11, -12); platelet activating factor acetyl hydrolase (PAF-AH); lipoprotein-associated phospholipase A2 (Lp-PLA2); von Willebrand Factor (vWF).

Proximal inflammatory markers are known to the person skilled in the art, and non-limiting examples include the following:
Pregnancy-associated polypeptide A (PAPP-A), intercellular adhesion molecules (e.g. ICAM-1, VCAM-1), IL-1-beta, IL-6, IL-18/IL-18b; TNF-alpha; myeloperoxidase (MPO); TF; monocyte chemoattractant protein 1 (MCP-1); P-selectin; E-selectin; matrix metalloproteinases (MMPs, e.g. MMP-1, -2, -3, -4, -5, -6, -7, -9, -10, -11, -12), platelet activating factor acetyl hydrolase (PAF-AH); lipoprotein-associated phospholipase A2 (Lp-PLA2); von Willebrand Factor (vWF).

Within the group of proximal markers, it is preferred if the latter is selected from matrix metalloproteinases MMPs, for example MMP-1, -2, -3, -4, -5, -6, -7, -9, -10, -11, -12, PAPP-A, and Lp-PLA2.

The preferred proximal inflammatory markers are PAPP-A, MMP-9 and Lp-PLA2.

The most preferred proximal inflammatory markers are PAPP-A and Lp-PLA2, in particular PAPP-A.

Proximal inflammatory markers are macromolecules situated upstream, i.e close to or at the ethiopathogenetic origin of the disease event. In particular, they are produced at the site of the coronary heart lesion, preferably the arterial plaque.

In contrast to proximal inflammatory markers, reactive inflammatory markers are produced downstream of or secondary to the disease event. For example, the acute-phase protein CRP (C-reactive protein) is produced by a distal organ (i.e. the liver) in response or reaction to chemokines or interleukins originating from the primary lesion site.

Examples of these so-called reactive inflammatory markers, include the following:
hsCRP, fibrinogen, serum amyloid A (SAA)
hsCRP is currently the most preferred inflammatory marker for risk stratification of acute coronary syndromes. However, hsCRP does not exert its diagnostic and predictive power as much as proximal inflammatory markers do.

Anti-inflammatory markers which can be used with the combination according to the present invention include IL-10.

Neurohumoral markers which can be used with the combination according to the present invention are: atrial natriuretic peptide (ANP); NT-pro atrial natriuretic peptide (NT-proANP); brain natriuretic peptide (BNP); NT-pro brain natriuretic peptide (NT-proBNP);

Preferred neurohumoral markers are:
BNP, NT-pro BNP.

The most preferred neurohumoral marker is NT-proBNP

Neurohumoral markers are in particular associated with patients having already suffered from MI (ST-elevated or non-ST-elevated) and being at risk for suffering from LVD and CHF.

Further inflammatory markers which can be used together with the marker combination according to the invention are selected from the group cited beforehand, namely the following: CRP, preferably hsCRP, fibrinogen, serum amyloid A (SAA), pregnancy-associated polypeptide A (PAPP-A), intercellular adhesion molecules (e.g. ICAM-1, VCAM-1), IL-1-beta, IL-6, IL-18/IL-18b; TNF-alpha; myeloperoxidase (MPO); TF; monocyte chemoattractant protein 1 (MCP-1); P-selectin; E-selectin; matrix metalloproteinases (MMPs, e.g. MMP-1, -2, -3, -4, -5, -6, -7, -9, -10, -11, -12), platelet activating factor acetyl hydrolase (PAF-AH); lipoprotein-associated phospholipase A2 (Lp-PLA2); vWF.

The further inflammatory marker will be selected such that it is different from the first (proximal) inflammatory marker used.

It is preferred if the further inflammatory marker is also a proximal marker (preferably Lp-PLA2); i.e. the marker combination PAPP-A/Lp-PLA2 is preferred over the marker combination PAPP-A/hsCRP.

Preferred combinations of sCD40L with a proximal inflammatory marker according to the present invention are the following:
sCD401, PAPP-A;
sCD40L, Lp-PLA2;
sCD40L, MMP-9.

The most preferred combination is sCD40L, PAPP-A.

Preferred combinations of sCD40L, a proximal inflammatory marker and a further inflammatory marker are the following:
sCD40L, PAPP-A, Lp-PLA2
sCD40L, PAPP-A, hsCRP
sCD40L, MMP-9, Lp-PLA2
sCD40L, MMP-9, hsCRP

The most preferred combination is sCD40L, PAPP-A, Lp-PLA2

Preferred combinations of sCD40L, a proximal inflammatory marker and an anti-inflammatory marker are the following:
sCD40L, PAPP-A, IL-10;
sCD40L, Lp-PLA2, IL-10;
sCD40L, MMP-9, IL-10.

The most preferred combination is sCD40L, PAPP-A, IL10.

Preferred combinations of sCD40L, a proximal inflammatory marker and a neurohumoral marker are the following:
sCD401, PAPP-A, NT-proBNP;
sCD40L, Lp-PLA2, NT-proBNP ;
sCD40L, MMP-9, NT-proBNP.

The most preferred combination is sCD40L, PAPP-A, NT-proBNP.

In case two markers further to the combination sCD40L and an inflammatory marker are used, it is preferred to use the combinations which have been designated as being preferred beforehand, and to combine these with the preferred markers from the groups of markers which combinable with the said combination.

Examples for such preferred combinations include the following:
When an individual has been analysed with the method according to the present invention for the risk of suffering from a future coronary heart event or if the individual has already suffered from a coronary heart event, said individual can be stratified for one or more certain therapeutic treatments. These can be selected from antibodies (monoclonal antibodies, polyclonal antibodies), small molecules, pharmacologically active compounds, i.e. anti-inflammatory and lipid-lowering drugs (e.g. statins), thrombolytic drugs (e.g. platelet antagonists), fibrinolytic drugs (e.g. heparin), revascularization therapy (e.g. PCTI (percutaneous therapeutic intervention), balloon dilatation, stenting, by-pass surgery).

The method according to the present invention permits a therapeutic treatment monitoring of the individual which is treated by said regimen.

In one embodiment of the present invention, the individual having suffered from or being at risk of suffering from a coronary heart event will be treated by administering an inhibitor or antagonist of the platelet glycoprotein IIb/IIIa receptor. Examples for such inhibitors include monoclonal or polyclonal antibodies, tirofiban, eptifibatide, and the like.

In a preferred embodiment of the present invention, the glycoprotein IIb/IIIa receptor inhibitor is an antibody, in particular the antibody known under the name Abciximab. Abciximab is a Fab antibody which is available under the name ReoPro from Centocor Europe BV.

In a further embodiment of the present invention, an anti-CD40L monoclonal antibody might be administered to the individual.

In a further embodiment of the present invention, an anti-inflammatory agent will be administered to the individual under surveillance. The anti-inflammatory agent can be an non-steroid anti-inflammatory agent or a steroid anti-inflammatory agent.

Examples for non-steroid anti-inflammatory agents include Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluormetholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminium; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salycilates; Sanguinarium Chloride; Seclazone; Sermetacin; Sudoxicam; Sulindac; Suprofen; Talmetacin; Talniflumate; Talosalate; Tebufelone; Tenidap; Tenidap Soidum; Tenoxicam; Tesicam; Tesimide; Tetrydamine; Tiopinac; Tixocortol Pivalate; Tolmetin; Tolmetin Sodium; Triclonide; Triflumidate; Zidometacin; Glucocorticoids; Zomepirac Sodium.

Examples for steroid anti-inflammatory agents include Glucocorticoids, for example dexamethasone, and further agents known to the person skilled in the art.

Further compound classes and compounds which can be administered to the individual under surveillance are: Heparin and derivates; Marcumar; rTpA: recombinant tissue plasminogen activator, thrombin receptor antagonists like aspirin and clopidogrel; insulin sensitizers (glitazones); calcium channel blockers; ACE inhibitors.

Further compound classes which can be administered to the individual under surveillance are statins.

Methods which can be used to determine the concentrations of the respective markers in a body fluid of the individual under examination are known to the person skilled in the art. These methods include microplate ELISA-based methods, fully-automated immunoassays (available for example on Elecsys™ analyzers for cTnT testing), CBA (an enzymatic Cobalt Binding Assay, available for example on Roche-Hitachi™ analyzers for IMA testing); latex agglutination assays (available for example on Roche-Hitachi™ analyzers for hsCRP testing

In the following, a general survey about the methods known to the skilled artisan will be given.

Peptides and polypeptides (proteins) can be measured in tissue, cell, and body fluid samples, i.e. preferably in vitro. It is preferred to measure the markers (proteins) in a body fluid.

Body fluids according to the present invention particularly include whole blood, blood serum, blood plasma, lymph, cerebral liquor, saliva, and urine. It is preferred to use whole blood, blood serum or plasma for the determination in the context of the present invention.

A tissue sample according to the present invention refers to any kind of tissue obtained from the dead or alive human or animal body. Tissue samples can be obtained by any method known to the person skilled in the art, for example by biopsy or curettage.

Methods to obtain cell samples include directly preparing single cells or small cell groups, dissociating tissue (e.g. using trypsin), and separating cells from body fluids, e.g. by filtration or centrifugation. Cells according to the present invention comprise also platelets and other non-nuclear cells, e.g. erythrocytes.

If necessary, the samples may be further processed. Particularly, nucleic acids, peptides or polypeptides may be purified from the sample according to methods known in the art, including filtration, centrifugation, or extraction methods such as chloroform/phenol extraction.

The person skilled in the art is familiar with different methods of determining (measuring) the concentration of a peptide or polypeptide (marker) in a sample (amount per volume). Measuring can be done indirectly, through measuring of cellular responses, the amount of bound ligands, labels, or enzymatic reaction products.

For measuring cellular responses, the sample or processed sample is added to a cell culture and an internal or external cellular response is measured. The cellular response may include the expression of a reporter gene or the secretion of a substance, e.g. a peptide, polypeptide, or a small molecule.

In a preferred embodiment of the present invention, the concentration of a ligand binding specifically to the peptide or polypeptide of interest (the marker) is determined. Binding according to the present invention includes both covalent and non-covalent binding.

A ligand according to the present invention can be any peptide, polypeptide, nucleic acid, or other substance binding to the peptide or polypeptide of interest. It is well known that peptides or polypeptides, if obtained or purified from the human or animal body, can be modified, e.g. by glycosylation. A suitable ligand according to the present invention may bind the peptide or polypeptide also via such sites.

Preferably, the ligand should bind specifically to the Marker to be determined. "Specific binding" according to the present invention means that the ligand should not bind substantially to ("cross-react" with) another peptide, polypeptide or substance present in the sample investigated. Preferably, the specifically bound protein or isoform should be bound with at least 3 times higher, more preferably at least 10 times higher and even more preferably at least 50 times higher affinity than any other relevant peptide or polypeptide.

Non-specific binding may be tolerable, particularly if the investigated peptide or polypeptide can still be distinguished and measured unequivocally, e.g. according to its size on a Western Blot, or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot).

For measurement of enzymatic reaction products, preferably the amount of substrate is saturating. The substrate may also be labeled with an detectable lable prior to the reaction. Preferably, the sample is contacted with the substrate for an adequate period of time. An adequate period of time refers to the time necessary for an detectable, preferably measurable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand.

Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

Enzymatically active labels include e.g. horseradish peroxidase, alkaline phosphatase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously.

Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon).

Typical radioactive labels include ³⁵S, ¹²⁵I, ³²P, ³³P and the like. A radioactive label can be detected by any method known and appropriate, e.g. a light-sensitive film or a phosphor imager.

Suitable measurement methods according the present invention also include electrochemiluminescence (electro-generated chemiluminescence), ELISA (enzyme-linked immunosorbent assay), sandwich enzyme immune tests, turbidimetry, nephelometry, latex-enhanced turbidimetry or nephelometry, or solid phase immune tests.

Preferred ligands include antibodies, nucleic acids, peptides or polypeptides, and aptamers, e.g. nucleic acid or peptide aptamers. Methods to such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

The term "antibody" as used herein includes both polyclonal and monoclonal antibodies, as well as fragments thereof, such as Fv, Fab and F(ab)₂ fragments that are capable of binding antigen or hapten. The present invention also includes "humanized" hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art.

Examples for ligands or reagents for measurement of peptides or polypeptides of particular interest include:
**sCD40L**
   ELISA (e.g. from R&D Systems, Wiesbaden, Germany or from BenderMedSystems, Vienna, Austria, and Alexis Biochemicals); antibodies, including FITC-tagged and FLAG-tagged antibodies are also available from Alexis (Grünberg, Germany), Ancell, and Immunotech (Hamburg, Germany); antigens from Abbott Pharmaceuticals, Abbott Park, Illinois; CD40-receptor.
**NT-proBNP:**
   one-step immunoassay based on electrochemiluminescence technology (Elecsys 2010 and E170 from Roche Diagnostics GmbH, Mannheim, Germany) is available
**PAPP-A:**
   PAPP-A can be measured by manual or semi-automated ELISA microplates and is available for example from IBL (Hamburg, Germany) or DSL (Webster, TX, USA)
**IL-10:**
   Manual ELISA (e.g. R&D Systems, Wiesbaden, Germany)
**MMP-9:**
   Manual ELISA (e.g. R&D Systems, Wiesbaden, Germany)
**Lp-PLA2:**
   Manual ELISA (e.g. diaDexus, USA)

In another preferred embodiment, the ligand, preferably chosen from the group consisting of nucleic acids, peptides, polypeptides, more preferably from the group consisting of nucleic acids, antibodies, or aptamers, is present on an array.

Said array contains at least one additional ligand, which may be directed against a peptide, polypeptide or a nucleic acid of interest. Said additional ligand may also be directed against a peptide, polypeptide or a nucleic acid of no particular interest in the context of the present invention. Preferably, ligands for at least three, preferably at least five, more preferably at least eight peptides or polypeptides of interest in the context of the present invention are contained on the array.

According to the present invention, the term "array" refers to a solid-phase or gel-like carrier upon which at least two compounds are attached or bound in one-, two- or three-dimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon.

The aforementioned array may include a bound ligand or at least two cells expressing each at least one ligand.

Methods of producing such arrays, for example based on solid-phase chemistry and photo-labile protective groups, are generally known (US 5,744,305). Such arrays can also be brought into contact with substances or substance libraries and tested for interaction, for example for binding or change of confirmation. Therefore, arrays comprising a peptide or polypeptide as defined above may be used for identifying ligands binding specifically to said peptides or polypeptides.

## Claims

1. A method for establishing a diagnosis if an individual has suffered from or a prognosis of an individual is at risk of suffering from a coronary heart event, which method comprises
- determining the concentration of sCD40L and a proximal inflammatory marker in a body fluid of the individual;
- comparing the values obtained with reference values; and
- establishing the diagnosis or the prognosis in respect to the said coronary heart event.

2. The method according to claim 1, wherein the proximal inflammatory marker is selected from PAPP-A, intercellular adhesion molecules, ICAM-1, VCAM-1, IL-1-beta, IL-6, IL-18/IL-18b; TNF-alpha; MPO; TF; MCP-1; P-selectin; E-selectin; matrix metalloproteinases; Lp-PLA2; von Willebrand Factor (vWF)..

3. The method according to claim 1, wherein the proximal inflammatory marker is selected from MMP-1, -2, -3, -4, -5, -6, -7, -9, -10, -11, -12, Lp-PLA2, and PAPP-A..

4. The method according to claim 1, wherein the proximal inflammatory marker is selected from PAPP-A, MMP-9 and Lp-PLA2.

5. The method according to claim 1, wherein the proximal inflammatory marker is PAPP-A.

6. The method according to any of the claims 1 to 5, wherein the combination is selected from the following: sCD40L, PAPP-A; sCD40L, Lp-PLA2; sCD40L, MMP-9.

7. The method according to any of claims 1 to 6, wherein the concentration of at least one marker further to CD40L and a proximal inflammatory marker is determined, with the said further marker being an anti-inflammatory marker.

8. The method according to claim 7, wherein the anti-inflammatory marker is IL-10.

9. The method according to any of claims 1 to 6, wherein the concentration of at least one marker further to sCD40L and a proximal inflammatory marker is determined, with the said further marker being a neurohumoral marker.

10. The method according to claim 9, wherein the neurohumoral marker is selected from ANP; NT-proANP; BNP; NT-proBNP.

11. The method according to claim 10, wherein the neurohumoral marker is NT-proBNP.

12. The method according to any of claims 1 to 6, wherein the concentration of at least one marker further to sCD40L and a proximal inflammatory marker is determined, with the said further marker being a further inflammatory marker different from the first inflammatory marker.

13. The method according to claim 12, wherein the further inflammatory marker is selected from CRP, hsCRP, fibrinogen, serum amyloid A (SAA), pregnancy-associated polypeptide A (PAPP-A), intercellular adhesion molecules (e.g. ICAM-1, VCAM-1), IL-1-beta, IL-6, IL-18/II,-18b; TNF-alpha; myeloperoxidase (MPO); procoagulant tissue factor (TF); monocyte chemoattractant protein 1 (MCP-1); P-selectin; E-selectin; matrix metalloproteinases (MMPs, e.g. MMP-1, -2, -3, -4, -5, -6, -7, -9, -10, -11, -12), platelet activating factor acetyl hydrolase (PAF-AH); lipoprotein-associated phospholipase A2 (Lp-PLA2); vWF.

14. The method according to claim 13, wherein the further inflammatory marker is a proximal inflammatory marker.

15. The method according to claim 13, wherein the further inflammatory marker is PAPP-A, Lp-PLA2 or MMP-9.

16. The method according to any of claims 1 to 15, wherein the concentration of at least two markers further to sCD40L and a proximal inflammatory marker is determined, with one further marker being selected from anti-inflammatory markers and the other further marker being selected from neurohumoral markers.

17. The method according to any of claims 1 to 16, wherein the individual is an individual presenting with acute coronary syndromes (ACS), chest pain or breathlessness, or an individual which does not show any symptoms related to a coronary heart disease.

18. The method according to claim 17, wherein the individual is an individual which does not show any symptoms related to a coronary heart disease

19. The method according to claim 17, wherein the ACS is one or more events selected from coronary heart disease (CHD); stable angina pectoris (SAP); unstable angina pectoris (UAP); acute coronary syndrome (ACS); acute myocardial infarction (AMI), both in cases of ST-elevation and non ST-elevation; left ventricular dysfunction (LVD); congestive heart failure (CHF).

20. The method according to claim 17, wherein the risk for CHD includes risk scores according to Framingham, Procam, patients with diabetes type I and II, diabetic patients with renal disorders, patients after an acute cardiovascular event, post myocardial infarction or ischemic stroke, patients with advanced and severe atherosclerosis, peripheral arteriovascular disease (PAD), hypercholesterolemia, hypertension, genetic predisposition, familial history of AMI or ischemic stroke, metabolic syndromes, obesity, ex-smokers, and other non-symptomatic patients without conventional risk factors.

21. The method according to any of claims 1 to 20, wherein the diagnosis or prognosis is employed in a therapy stratification or a treatment monitoring.

22. The method according to claim 21, wherein a treatment monitoring is established after administering to the individual a pharmaceutically active substance selected from antibodies, small molecules, and pharmacologically active proteins.
